(19) Europäisches Patentamt
European Patent Office
Office européen des brevets

(11) Veröffentlichungsnummer: **0 145 660**
**B1**

(12) **EUROPÄISCHE PATENTSCHRIFT**

(45) Veröffentlichungstag der Patentschrift: **18.04.90**

(51) Int. Cl.$^5$: **C 07 D 251/70**, A 01 N 43/68

(21) Anmeldenummer: **84810577.1**

(22) Anmeldetag: **28.11.84**

# (54) Schädlingsbekämpfungsmittel.

(30) Priorität: **02.12.83 CH 6466/83**
**02.11.84 CH 5267/84**

(43) Veröffentlichungstag der Anmeldung:
**19.06.85 Patentblatt 85/25**

(45) Bekanntmachung des Hinweises auf die Patenterteilung:
**18.04.90 Patentblatt 90/16**

(84) Benannte Vertragsstaaten:
**AT BE CH DE FR IT LI NL SE**

(56) Entgegenhaltungen:
**US-A-3 520 974**
**US-A-4 225 598**

**JOURNAL OF ECONOMIC ENTOMOLOGY, Band 61, Nr. 6, Dezember 1968, Seiten 1621-1632, Calvere Road, College Park, MD 20740-3493, US; G.C. LABRECQUE et al.: "Substituted melamines as chemosterilants of house flies"**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Gehret, Jean-Claude, Dr.**
**Im Aeschfeld 12**
**CH-4147 Aesch (CH)**
Erfinder: **Kristiansen, Odd, Dr.**
**CH-4313 Möhlin**
**CH-4313 Mahlin (CH)**

**EP 0 145 660 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft neue substituierte Acylamino-s-triazin-Verbindungen und ihre Salze, Verfahren zu ihre Herstellung und ihre Verwendung zur Bekämpfung von Schadinsekten und Ektoparasiten.

Die neuen Verbindungen haben die allgemeine Formel I

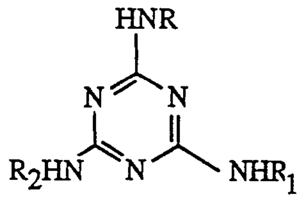

(I)

in welcher

R $C_1$—$C_4$-Alkyl oder $C_3$—$C_6$-Cycloalkyl,

$R_1$ Wasserstoff oder gegebenenfalls durch Halogen oder $C_1$—$C_4$-Alkoxy substituiertes Alkanoyl mit 2 bis 12 Kohlenstoffatomen oder $C_3$—$C_6$-Cycloalkyl-carbonyl und

$R_2$ gegebenenfalls durch Halogen oder $C_1$—$C_4$-Alkoxy substituiertes Alkanoyl mit 2 bis 12 Kohlenstoffatomen oder $C_3$—$C_6$-Cycloalkyl-carbonyl bedeuten, sowie deren Säureadditionssalze, mit der Massgabe, dass

a) wenn R Methyl und $R_1$ Wasserstoff ist, $R_2$ nicht Acetyl ist, und

b) wenn R $C_1$—$C_4$-Alkyl und $R_1$ Alkanoyl mit 2 bis 4 Kohlenstoffatomen ist, $R_2$ nicht Alkanoyl mit 2 bis 4 Kohlenstoffatomen ist. Alkyl als Rest R oder als Bestandteil von $R_1$ und $R_2$ kann geradkettig oder verzweigt sein. Beispiele dafür sind Methyl, Aethyl, die Isomeren von Propyl, Butyl, Pentyl, Hexyl, Heptyl, Octyl, Nonyl, Decyl und Undecyl. Cyclische Alkyle als R oder als Bestandteil von $R_1$ und $R_2$ sind Cyclopropyl, Cyclobutyl, Cyclopentyl und Cyclohexyl.

Als gleiche oder verschiedene Halogen-Substituenten der Alkanoylreste und Cycloalkyl-carbonylreste von $R_1$ und $R_2$ sind Fluor, Chlor, Brom oder Jod zu verstehen.

Alkoxy-Reste als Alkanoylsubstituenten oder Substituenten der Cycloalkyl-carbonylreste sind Methoxy, Aethoxy oder die Isomeren von Propoxy und Butoxy.

Wegen ihrer besonders vorteilhaften Wirkung sind diejenigen Verbindungen der Formel I bevorzugt, in welchen

$R_1$ Wasserstoff oder gegebenenfalls durch Halogen substituiertes Alkanoyl mit 2 bis 12 Kohlenstoffatomen oder $C_3$—$C_6$-Cycloalkyl-carbonyl und

$R_2$ gegebenenfalls durch Halogen substituiertes Alkanoyl mit 2 bis 12 Kohlenstoffatomen oder $C_3$—$C_6$-Cycloalkyl-carbonyl bedeuten und

R die unter Formel 1 angegebenen Bedeutungen besitzt, sowie deren Säureadditionssalze, wobei die Massgaben a) und b) gelten, oder in welchen

$R_1$ Wasserstoff oder gegebenenfalls durch $C_1$—$C_4$-Alkoxy substituiertes Alkanoyl mit 2 bis 12 Kohlenstoffatomen oder $C_3$—$C_6$-Cycloalkyl-carbonyl und

$R_2$ gegebenenfalls durch $C_1$—$C_4$-Alkoxy substituiertes Alkanoyl mit 2 bis 12 Kohlenstoffatomen oder $C_3$—$C_6$-Cycloalkyl-carbonyl bedeuten und

R die unter Formel I angegebenen Bedeutungen besitzt, sowie deren Säureadditionssalze, wobei die Massgaben a) und b) gelten, oder in welchen

R Cyclopropyl oder iso-Propyl,

$R_1$ Wasserstoff oder gegebenenfalls durch Chlor substituiertes Alkanoyl mit 2 bis 12 Kohlenstoffatomen oder $C_3$—$C_6$-Cycloalkyl-carbonyl und

$R_2$ gegebenenfalls durch Chlor substituiertes Alkanoyl mit 2 bis 12 Kohlenstoffatomen oder $C_3$—$C_6$-Cycloalkyl-carbonyl bedeuten, sowie deren Säureadditionssalze, mit der Massgabe, dass, wenn R Isopropyl und $R_1$ Alkanoyl mit 2 bis 4 Kohlenstoffatomen ist, $R_2$ nicht Alkanoyl mit 2 bis 4 Kohlenstoffatomen ist, oder in welchen

R Cyclopropyl oder iso-Propyl,

$R_1$ Wasserstoff oder gegebenenfalls durch Methoxy oder Aethoxy substituiertes Alkanoyl mit 2 bis 12 Kohlenstoffatomen oder $C_3$—$C_6$-Cycloalkyl-carbonyl und

$R_2$ gegebenenfalls durch Methoxy oder Aethoxy substituiertes Alkanoyl mit 2 bis 12 Kohlenstoffatomen oder $C_3$—$C_6$-Cycloalkyl-carbonyl bedeuten, sowie deren Säureadditionssalze, mit der Massgabe, dass, wenn R Isopropyl und $R_1$ Alkanoyl mit 2 bis 4 Kohlenstoffatomen ist, $R_2$ nicht Alkanoyl mit 2 bis 4 Kohlenstoffatomen ist, oder in welchen

R Cyclopropyl oder iso-Propyl,

$R_1$ Wasserstoff und

$R_2$ gegebenenfalls durch Chlor substituiertes Alkanoyl mit 2 bis 12 Kohlenstoffatomen oder $C_3$—$C_6$-Cycloalkyl-carbonyl bedeuten, sowie deren Säureadditionssalze,

oder insbesondere solche Verbindungen der Formel I, in welchen
R Cyclopropyl,
$R_1$ Wasserstoff und
$R_2$ Alkanoyl mit 2 bis 12 Kohlenstoffatomen oder $C_3$—$C_6$-Cycloalkyl-carbonyl bedeuten, sowie deren Säureadditionssalze.

Folgende Einzelverbindungen sind bevorzugt:
2-Cyclopropylamino-4-acetylamino-6-amino-1,3,5-triazin,
2-Cyclopropylamino-4-methoxyacetylamino-6-amino-1,3,5-triazin,
2-Cyclopropylamino-4-propanoylamino-6-amino-1,3,5-triazin,
2-Cyclopropylamino-4-butanoylamino-6-amino-1,3,5-triazin und
2-Cyclopropylamino-4-(2-methylpropanoylamino)-6-amino-1,3,5-triazin.

Unter dem Begriff Salze der Acylamino-s-triazin-Verbindungen der Formel I sind Additionssalze anorganischer und organischer Säuren zu verstehen.

Beispiele anorganischer Säuren sind Chlorwasserstoffsäure, Bromwasserstoffsäure, Jodwasserstoffsäure, Schwefelsäure, Phosphorsäure, phosphorige Säure, Salpetersäure.

Beispiele organischer Säuren sind Trifluoressigsäure, Trichloressigsäure, Ameisensäure, Oxalsäure, Bernsteinsäure, Maleinsäure, Milchsäure, Glycolsäure, Aconitsäure, Zitronensäure, Benzoesäure, Benzolsulfonsäure, Methansulfonsäure.

Die Verbindungen der Formel I werden hergestellt, indem man Verbindungen der Formel II

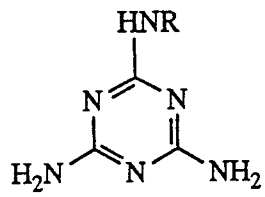

(II)

mit Verbindungen der Formel IIIa

$$R_2'—CO—Hal \qquad (IIIa)$$

oder der Formel IIIa'

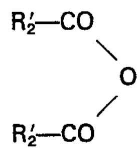

(IIIa')

zu Verbindungen der Formel Ia

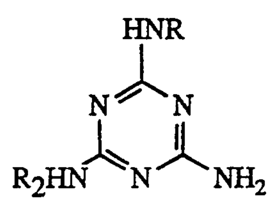

(Ia)

und gegebenenfalls Verbindungen der Formel Ia mit Verbindungen der Formel IIIb

$$R_1''—CO—Hal \qquad (IIIb)$$

oder der Formel IIIb'

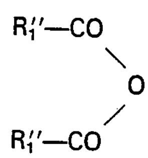

(IIIb')

zu Verbindungen der Formel Ib

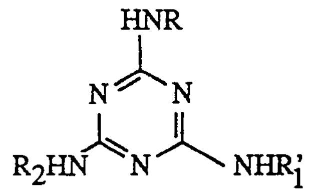

(Ib)

umsetzt, in welchen $R_1'$ und $R_2$ unabhängig voneinander gegebenenfalls durch Halogen oder $C_1$—$C_4$-Alkoxy substituiertes Alkanoyl mit 2 bis 12 Kohlenstoffatomen oder $C_3$—$C_6$-Cycloalkyl-carbonyl und $R_1''$ und $R_2'$ unabhängig voneinander gegebenenfalls durch Halogen oder $C_1$—$C_4$-Alkoxy substituiertes $C_1$—$C_{11}$-Alkyl oder $C_3$—$C_6$-Cycloalkyl darstellen und Hal Halogen, vorzugsweise Chlor, bedeutet und R die unter Formel I angegebenen Bedeutungen besitzt.

Das vorstehend angegebene Verfahren wird unter normalem Druck in inerten Lösungs- oder Verdünnungsmitteln in Gegenwart einer Base bei Temperaturen von 0 bis 120°C, vorzugsweise 40 bis 80°C, durchgeführt.

Als Lösungs- und Verdünnungsmittel kommen beispielsweise in Frage:

Alkane wie n-Pentan sowie seine Homologen einschliesslich der Isomeren bis zum n-Heptadecan;

Aether wie Diäthyläther, Dipropyläther, Dibutyläther, Dimethoxyäthan, Dioxan oder Tetrahydrofuran;

Chlorierte Kohlenwasserstoffe wie Chloroform, Methylenchlorid, Tetrachlorkohlenstoff oder Chlorbenzol;

Aromatische Kohlenwasserstoffe wie Benzol, Toluol oder die Xylole.

Darüber hinaus können weitere inerte Lösungs- und Verdünnungsmittel in dem Verfahren Anwendung finden.

Als Base eignen sich beispielsweise Alkylamine wie Triäthylamin oder Diisopropyläthylamin sowie ferner Pyridin oder N-Methylpyrrolidon.

Das vorstehend beschriebene Verfahren zur Herstellung der Verbindungen der Formel I ist im Prinzip aus der Literatur bekannt (vgl. The Chemistry of Heterocyclic Compounds, Vol. 13: s-Triazines and Derivatives, Interscience Publishers Inc., New York, 1959).

Die Ausgangsstoffe der Formeln II, IIIa, IIIa', IIIb und IIIb' sind bekannt und lassen sich mit entsprechend bekannten Verfahren herstellen.

Diamino- und Triamino-Derivate von s-Triazinen sind als Chemosterilantien für adulte Stubenfliegen (Musca domestica) aus der US-Patentschrift Nr. 3.189.521 bekannt.

Die insektenchemosterilisierende Wirkung von 2,4,6-Triamino-s-triazinderivaten (Melamin-Derivate) wird ferner von S. Nagasawa et al., Botyu-Kagaku *39* (4), 105 (1974) beschrieben. A. B. Borkovec und A. B. DeMilo (J. Med. Chem. *10* (5), 457 (1967) sowie G. C. LaBrecque, R. L. Fye, A. B. DeMilo und A. B. Borkovec (J. Econ. Entomol. *61* (6), 1621 (1968) beschreiben des weiteren die chemosterilisierende Wirkung von u.a. 2-Cyclohexylamino-4,6-diamino-s-triazin, 2-Cyclohexylamino-4,6-dihexylamino-s-triazin und 2,4,6-Triscyclohexylamino-s-triazin sowie ihrer Salze auf adulte Stubenfliegen (Musca domestica). Weiterhin ist die insektizide Wirkung von Cyclopropylamino-triazin-Derivaten aus der USA—Patentschrift 4,225,598 bekannt.

Es wurde nun überraschenderweise festgestellt, dass die Verbindungen der Formel I eine ausgeprägte larvizide Wirksamkeit gegen Insektenlarven, vorzugsweise Dipterenlarven, ausüben. Im Gegensatz zu den oben erwähnten Insektenchemosterilantien wirken die Verbindungen der Formel I vor allem auf die juvenilen Stadien der Insekten. Die Wirkung besteht in einem Absterben der Eilarven oder in der Verhinderung des Schlüpfens von Adulten aus den Puppen. Die Wirkung der Verbindungen der Formel I ist nicht mit der Wirkungsweise von klassischen Insektiziden, Chemosterilantien oder Juvenilhormonanalogen zu vergleichen.

Die Wirkstoffe der Formel I werden zur Bekämpfung von tierischen Ektoparasiten und von Hygieneschädlingen vorzugsweise aus der Ordnung Diptera und den Familien Culicidae, Simuliidae, Tipulidae, Muscidae und Calliphoridae eingesetzt. Als besonders wirksam erweisen sich dabei die Verbindungen der Formel I gegen Larven der zur Familie Calliphoridae gehörenden Blowfly (Lucilia sericata und Lucilia curpina) sowie gegen Fliegen- und Mückenlarven.

Weiterhin sind die Verbindungen der Formel I wirksam gegen Vertreter der Ordnungen Siphonaptera (z.B. blutsaugende Flöhe).

Neben ihrer Wirkung gegen Mücken und Fliegen, wie z.B. Aëdes aegypti und Musca domestica, können Verbindungen der Formel I auch zur Bekämpfung von Pflanzenschädigenden Frassinsekten in Zier- und Nutzpflanzungen, insbesondere in Reiskulturen (z.B. gegen Nilaparvata lugens und Laodelphax striatellus) erfolgreich eingesetzt werden.

In ihrer Aktivität zeigen die erfindungsgemässen Verbindungen eine Wirkungsbreite, die über das Larvenstadium hinausgehend auch die übrigen Entwicklungsstadien der Parasiten sowie die Ablage fertiler Eier umfasst.

Darüber hinaus zeichnen sich die Verbindungen der Formel I in völlig überraschender Weise durch eine biologische Langzeitwirkung aus, die ein besonderes Merkmal dieser Verbindungen darstellt. Diese

prolongierende Wirkungsweise kann sich je nach Anwendungsart über einen Zeitraum von mehr als 3 Monaten erstrecken, was gegenüber bekannten Präparaten vielfältige Vorteile bietet.

Beim Stall-hygienischen Einsatz der erfindungsgemässen Wirkstoffe wird dadurch beispielsweise eine äusserst niedrige Anwendungsfrequenz erreicht, sodass in gemässigten Klimazonen mit 3-monatiger Sommersaison eine einmalige Anwendung genügt, um eine durch die klimatischen Bedingungen normalerweise begünstigte Entwicklung der schädlichen Dipterenlarven in den Stallungen nachhaltig zu hemmen.

Bei der Behandlung von Weidetieren mit den erfindungsgemässen Verbindungen, z.B. mittels Viehbäder, Aufguss-Methoden oder Sprühgänge, wird durch die überraschende Adhäsionswirkung der Aktivsubstanzen ein lang anhaltender toxischer Effekt gegen Ektoparasiten, wie beispielsweise schädlichen Dipteren, auf der Haut und dem Fell der Tiere erzielt. Ein frühzeitiges Aus- oder Abwaschen der auf die Oberfläche der Nutztiere applizierten Wirkstoffe durch ablaufendes Regenwasser kann somit verhindert werden.

Der besondere Vorteil der verlängerten Wirkung der Verbindungen der Formel I wirkt sich vor allem bei der oralen Verabreichung an Nutztiere aus. Bei diesem Anwendungsverfahren entfalten die Wirkstoffe vor allem in den aus dem Verdauungstrakt ausgeschiedenen Fäkalien eine nachhaltige und langandauernde insektizide Aktivität. Dadurch kann der Befall mit schädlichen Insekten, insbesondere Dipteren, bereits vor dem Auftreten der Schädlinge in der Umgebung der Tiere, wie Stallungen, Gehegen und Weide, verhindert werden, weil die aus den abgelegten Eiern schlüpfenden Dipterenlarven sofort abgetötet werden. Bei dieser speziellen Anwendung ist besonders bedeutsam, dass sich die Verbindungen der Formel I aufgrund ihrer strukturellen Eigenschaften gegenüber Warmblütern physiologisch indifferent verhalten. Diese Methode der gezielten Bekämpfung der Proliferation der Insekten ist bedeutend effizienter und gleichzeitig ökonomischer als die üblichen grossflächigen Desinfektionen von Stallungen und Gehegen.

Zur Bekämpfung der Schädlinge werden die erfindungsgemässen Verbindungen der Formel I sowohl allein als auch in Form von Mitteln eingesetzt, welche noch geeignete Trägerstoffe und Zuschlagstoffe oder Gemische solcher Stoffe enthalten. Geeignete Träger- und Formulierungshilfsstoffe können fest oder flüssig sein und entsprechen den in der Formulierungstechnik üblichen Stoffen, wie z.B. natürlichen oder regenerierten Stoffen, Lösungs-, Dispergier-, Netz-, Haft-, Verdickungs- oder Bindemitteln.

Zur Applikation werden die Verbindungen der Formel I zu Stäubemitteln, Emulsionskonzentraten, Granulaten, Dispersionen, Sprays, zu Lösungen oder Aufschlämmungen in üblichen Formulierungen, die in der Applikationstechnik zum Allgemeinwissen gehören, verarbeitet.

Die Herstellung der erfindungsgemässen Mittel erfolgt in an sich bekannter Weise durch inniges Vermischen und/oder Vermahlen von Wirkstoffen der Formel I mit geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden.

Feste Aufarbeitungsformen: Stäubemittel, Streumittel, Granulate (Umhüllungsgranulate, Imprägnierungsgranulate und Homogengranulate).

Flüssige Aufarbeitungsformen:
a) in Wasser dispergierbare Wirkstoffkonzentrate: Sritzpulver (wettable powder), Pasten, Emulsionen;
b) Lösungen: Aufguss, Sprühmittel.

Der Gehalt an Workstoff in den vorstehend beschriebenen Applikationsformen liegt zwischen 0,1 und 95,0 Gew.%, vorzugsweise zwischen 1 und 80 Gew.%.

Die erfindungsgemässen Verbindungen der Formel I sind als Wirkstoffe von Mitteln durch die verschiedenen Zubereitungsformen in vielfältiger Weise zur Bekämpfung von Parasiten auf oder in der Umgebung von Tieren, z.B. in Tierstallungen, geeignet. So können sie beispielsweise in Viehbädern (cattle dips), Sprühgängen (spray races), Aufgusslösungen (pour-on) oder Handsprühmitteln angewendet werden. Ferner sind sie mit gutem Erfolg für die Behandlung von tierischen Fäkalien mittels der "Feed-through"-Methode und die stall-hygienische Mistbehandlung einsetzbar.

## Beispiel 1

Herstellung von 2-Cyclopropylamino-4-heptanoylamino-6-amino-1,3,5-triazin

Zu einer Suspension von 33,2 g 2,4-Diamino-6-cyclopropylamino-1,3,5-triazin und 30,3 g Triäthylamin in 800 ml Tetrahydrofuran werden bei 60°C langsam 50,9 g Oenanthsäureanhydrid gelöst in 100 ml Tetrahydrofuran zugetropft. Das Reaktionsgemisch wird anschliessend über Nacht bei 60°C gerührt, mit Aktivkohle versetzt und dann heiss filtriert. Das Filtrat wird eingeengt und mit Isopropanol versetzt. Nach Filtrieren und Trocknen erhält man das Produkt als weisses kristallines Pulver mit einem Schmelzpunkt von 195—197°C.

Beispiel 2

Herstellung von 2,4-Bis-acetylamino-6-cyclopropylamino-1,3,5-triazin

Zu einer Suspension von 12,45 g 2,4-Diamino-6-cyclopropylamino-1,3,5-triazin in 100 ml Pyridin werden 12 g Acetylchlorid langsam zugetropft. Anschliessend wird das Reaktionsgemisch 1 Stunde bei 55°C und dann 16 Stunden bei Raumtemperatur gerührt. Das Produkt erhält man nach Abfiltrieren und Waschen des Filterrückstandes mit Wasser und anschliessendem Trocknen als weisses kristallines Pulver mit einem Schmelzpunkt von 267—269°C.

Beispiel 3

Herstellung von 2-Cyclopropylamino-4-acetylamino-6-amino-1,3,5-triazin

Zu einer Suspension von 49,8 g 2,4-Diamino-6-cyclopropylamino-1,3,5-triazin und 45,5 g Triaethylamin in 1200 ml Tetrahydrofuran werden bei ca. 60°C langsam (in ca. 6 Stdn.) 31,7 g Acetanhydrid gelöst in 100 ml Tetrahydrofuran zugetropft. Das Reaktionsgemisch wird anschliessend 18 Stdn. unter Rückfluss gekocht, heiss filtriert und anschliessend bis zum Einsetzen der Kristallisation eingeengt. Nach Zugabe von ca. 300 ml Diaethylaether wird das Produkt als weisses kristallines Pulver abfiltriert. Es hat einen Schmelzpunkt von 190—195°C.

Beispiel 4

Herstellung von 2-Cyclopropylamino-4-propanoylamino-6-amino-1,3,5-triazin-HCl

2,2 g 2-Cyclopropylamino-4-propanoylamino-6-amino-1,3,5-triazin werden in 6 ml 2N HCl gelöst und 5 Min. bei Raumtemperatur gerührt. Nach Einengen und Trocknen erhält man das Produkt als weisses kristallines Pulver mit einem Schmelzpunkt von 210—212°C.

Beispiel 5

Herstellung von 2-Methoxyacetylamino-4-Cyclopropylamino-6-amino-1,3,5-triazin

Zu einer Suspension von 8,3 g 2,4-Diamino-6-cyclopropylamino-1,3,5-triazin und 7,1 g Triäthylamin in 250 ml Tetrahydrofuran werden bei ca. 60°C langsam (in ca. 5 Stdn.) 8,9 g Methoxy-acetanhydrid gelöst in 30 ml Tetrahydrofüran zugetropft. Das Reaktionsgemisch wird anschliessend 18 Stdn. unter Rückfluss gekocht, heiss filtriert und anschliessend bis zum Einsetzen der Kristallisation eingeengt. Nach Zugabe von ca. 300 ml Diaethylaether wird das Produkt als weisses kristallines Pulver abfiltriert. Es hat einen Schmelzpunkt von 152—157°C.

Beispiel 6

Herstellung von 2,4-Bis-methoxyacetylamino-6-cyclopropyl-amino-1,3,5-triazin

Zu einer Suspension von 5,0 g 2,4-Diamino-6-cyclopropylamino-1,3,5-triazin in 50 ml Pyridin werden 7,2 g Methoxyacetylchlorid langsam zugetropft. Anschliessend wird das Reaktionsgemisch 1 Stunde bei 55°C und dann 16 Stunden bei Raumtemperatur gerührt. Das Produkt erhält man nach Abfiltrieren und Waschen des Filterrückstandes mit Wasser und anschliessendem Trocknen als weisses kristallines Pulver mit einem Schmelzpunkt von 175—180°C.

Tabelle 1

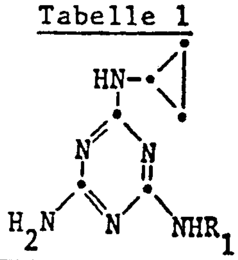

| Nr. | $R_1$ | Salz | Fp [°C] |
|---|---|---|---|
| 1 | $-CO-CH_3$ | - | 190-195 |
| 2 | $-CO-CH_3$ | HCl | 245-247 |
| 3 | $-CO-C_2H_5$ | - | 226-229 |
| 4 | $-CO-C_2H_5$ | HCl | 210-212 |
| 5 | $-CO-C_3H_7(n)$ | - | 216-218 |
| 6 | $-CO-C_3H_7(n)$ | HCl | 95-100 (amorph) |
| 7 | $-CO-C_3H_7(i)$ | - | 232-236 |
| 8 | $-CO-C_3H_7(i)$ | HCl | 155-160 |
| 9 | $-CO-C_4H_9(t)$ | - | 80-85 (amorph) |
| 10 | $-CO-C_4H_9(t)$ | HCl | 60-65 (amorph) |
| 11 | $-CO-C_4H_9(n)$ | - | 182-183 |
| 12 | $-CO-C_4H_9(n)$ | HCl | 133-138 |
| 13 | $-CO-C_6H_{13}(n)$ | - | 195-197 |
| 14 | $-CO-C_6H_{13}(n)$ | HCl | 135 |
| 15 | $-CO-C_{11}H_{23}(n)$ | - | 175-177 |
| 16 | $-CO-C_{11}H_{23}(n)$ | HCl | 163 |
| 17 | -CO- 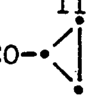 | - | 246-250 |
| 18 | -CO- 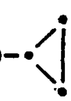 | HCl | 232-234 |
| 19 | $-CO-CH_2-Cl$ | | |
| 20 | $-CO-C_2H_4-Cl$ | | |
| 21 | $-CO-CH(Cl)-CH_3$ | | |
| 22 | $-CO-C_3H_{6(n)}-Cl$ | | |
| 23 | $-CO-CH(Cl)-C_2H_5$ | | |
| 24 | $-CO-CH_2-OCH_3$ | | |
| 25 | $-CO-C_2H_4-OCH_3$ | | |

Tabelle 2

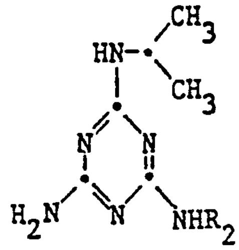

| Nr. | $R_2$ | Salz | Fp [°C] |
|---|---|---|---|
| 1 | $-CO-CH_3$ | - | 202-204 |
| 2 | $-CO-CH_3$ | HCl | 80-90 (amorph) |
| 3 | $-CO-C_3H_7(n)$ | - | 194-198 |
| 4 | $-CO-C_3H_7(n)$ | HCl | 75-85 (amorph) |
| 5 | $-CO-CH_2-Cl$ | | |
| 6 | $-CO-C_2H_4-Cl$ | | |
| 7 | $-CO-CH(Cl)-CH_3$ | | |
| 8 | $-CO-C_3H_{6(n)}-Cl$ | | |
| 9 | $-CO-CH(Cl)-C_2H_5$ | | |
| 10 | $-CO-CH_2-OCH_3$ | | |
| 11 | $-CO-C_2H_4-OCH_3$ | | |

Tabelle 3

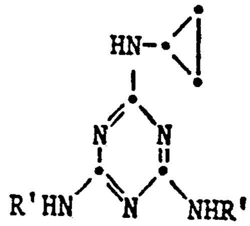

| Nr. | R' | Salz | Fp[°C] |
|---|---|---|---|
| 1 | $-CO-C_2H_5$ | - | 263-264 |
| 2 | $-CO-CH_3$ | - | 275 |
| 3 | $-CO-C_5H_{11}$ | - | 198-200 |
| 4 | -CO- 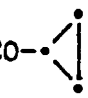 | - | 273-276 |
| 5 | -CO- 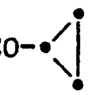 | HCl | 190 (Zers.) |
| 6 | $-CO-C_{11}H_{23}$ | - | 166-168 |
| 7 | $-CO-(CH_2)_3-Cl$ | - | 193-194 |
| 8 | $-CO-C_4H_9(t)$ | - | 65-70 (amorph) |
| 9 | $-CO-C_4H_9(t)$ | HCl | 73-78 (amorph) |
| 10 | $-CO-CH_2-Cl$ | | |
| 11 | $-CO-C_2H_4-Cl$ | | |
| 12 | $-CO-CH(Cl)-CH_3$ | | |
| 13 | $-CO-C_3H_{6(n)}-Cl$ | | |
| 14 | $-CO-CH(Cl)-C_2H_5$ | | |
| 15 | $-CO-CH_2-OCH_3$ | | |
| 16 | $-CO-C_2H_4-OCH_3$ | | |

Tabelle 4

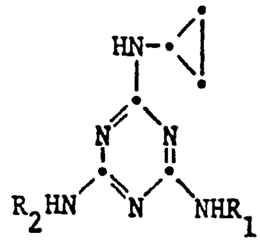

| Nr. | $R_1$ | $R_2$ | Salz | Fp. [°C] |
|---|---|---|---|---|
| 1 | $-CO-CH_3$ | $-CO-C_2H_5$ | - | 259-262 |
| 2 | $-CO-CH_3$ | $-CO-C_2H_5$ | HCl | 155 (Zers.) |
| 3 | $-CO-CH_3$ | $-CO-C_3H_7$(n) | - | 232-233 |
| 4 | $-CO-CH_3$ | $-CO-C_3H_7$(n) | HCl | 215-217(Zers.) |
| 5 | $-CO-C_2H_5$ | $-CO-C_3H_7$(n) | - | 242-244 |
| 6 | $-CO-C_2H_5$ | $-CO-C_3H_7$(n) | HCl | 217-219(Zers.) |
| 7 | $-CO-C_3H_7$(n) | $-CO-C_6H_{13}$(n) | - | 180-182 |
| 8 | $-CO-C_3H_7$(n) | $-CO-C_6H_{13}$(n) | HCl | 120-124 |
| 9 | $-CO-CH_3$ | $-CO-CH_2-Cl$ | | |
| 10 | $-CO-CH_3$ | $-CO-CH_2H_4-Cl$ | | |
| 11 | $-CO-CH_3$ | $-CO-CH(Cl)-CH_3$ | | |
| 12 | $-CO-C_2H_5$ | $-CO-C_3H_{6(n)}-Cl$ | | |
| 13 | $-CO-C_2H_5$ | $-CO-CH(Cl)-C_2H_5$ | | |
| 14 | $-CO-CH_3$ | $-CO-CH_2-OCH_3$ | | |
| 15 | $-CO-CH_3$ | $-CO-C_2H_4-OCH_3$ | | |

Beispiel 7

Wirkung gegen Lucilia sericata

Frisch abgelegte Eier der Schmeissfliege L. sericata werden in kleinen Portionen (30—50 Eier) in Reagenzgläser gegeben, in denen zuvor 4 ml Nährmedium mit 1 ml Testlösung in der für die Endkonzentration notwendigen Zwischenverdünnung vermischt worden sind. Nach der Beimpfung des Kulturmediums werden die Testgläser mit einem Wattestopfen verschlossen und im Brutschrank bei 30°C über 4 Tage bebrütet. In dem zum Vergleich unbehandelten Medium entwickeln sich bis zu diesem Zeitpunkt ca. 1 cm lange Larven (Stadium 3). Ist eine Substanz aktiv, so sind die Larven zu diesem Zeitpunkt entweder tot oder moribund und deutlich zurückgeblieben. Der Versuch wird simultan mit Konzentrationen von 10—0.01 ppm durchgeführt. Die Wirksamkeit wird gemessen an der niedrigsten noch voll wirksamen Konzentration (LC 100).

Der Test erfasst sowohl durch Kontakt als auch als Frassgift wirksame Substanzen. Auch Repellenz wird berücksichtigt, da die Larven aus dem Medium auswandern und verhungern.

Geprüfte verbindungen Nr. 1—8, 10—12 und 14 aus Tabelle 1 und Nr. 1 aus Tabelle 2 sind bei Konzentrationen von 0.1 bis 0.5 ppm voll worksam. Geprüfte Verbindungen 9, 13 und 16 aus Tabelle 1 und Verbindung Nr. 9 aus Tabelle 3 haben bei Konzentrationen von 1 bis 2.5 ppm volle Wirkung.

Beispiel 8

Wirkung gegen Lucilia cuprina

Frisch abgelegte Eier der Schmeissfliege L. cuprina werden in kleinen Portionen (30—50 Eier) in Reagenzgläser gegeben, in denen zuvor 4 ml Nährmedium mit 1 ml Testlösung in der für die Endkonzentration notwendigen Zwischenverdünnung vermischt worden sind. Nach der Beimpfung des Kulturmediums werden die Tesgläser mit einem Wattestopfen verschlossen und im Brutschrank bei 30° C über 4 Tage bebrütet.

Im dem zum Vergleich unbehandelten Medium entwickeln sich bis zu diesem Zeitpunkt ca. 1 cm lange Larven (Stadium 3). Ist eine Substanz aktiv, so sind die Larven zu diesem Zeitpunkt entweder tot oder moribund und deutlich zurückgeblieben. Der Versuch wird simultan mit Konzentrationen von 10—0.01 ppm durchgeführt. Die Wirksamkeit wird gemessen an der niedrigsten noch voll wirksamen Konzentration (LC 100).

Der Test erfasst sowohl durch Kontakt als auch als Frassgift wirksame Substanzen. Auch Repellenz wird berücksichtigt, da die Larven aus dem Medium auswandern und verhungern.

Geprüfte Verbindungen Nr. 1—8, 10—12 und 14 aus Tabelle 1 sind bei Konzentrationen von 0.01 bis 0.5 ppm voll worksam. Geprüfte Verbindungen Nr. 9, 13 und 16 aus Tabelle 1 und Verbindungen Nr. 1 und 9 aus Tabelle 3 haben bei Konzentrationen von 1 bis 2.5 ppm volle Wirkung.

Beispiel 9

Wirkung gegen Aedes aegypti

Auf die Oberfläche von 150 ml Wasser, dass sich in einem Behälter befindet, wird so viel einer 0,1%igen acetonischen Lösung des Wirkstoffes pipettiert, dass Konzentrationen von je 10, 5 und 1 ppm erhalten werden. Nach Verdunsten des Acetons wird der Behälter mit 30—40 3-tägigen Aëdes-Larven beschickt. Nach 1, 2 und 5 Tagen wird die Mortalitätsrate ermittelt.

Verbindungen gemäss den Beispielen 1—6 und den Tabellen 1—4 bewirken bei 1.5 oder 10 ppm 100% Abtötung.

Beispiel 10

Wirkung gegen Nilaparvata lugens (Nymphen) und Laodelphax striatellus

Der Test wird an wachsenden Reis-Pflanzen durchgeführt. Dazu werden jeweils 4 Pflanzen (Dicke des Stengels 8 mm; Höhe ca. 20 cm) in Töpfe (Durchmesser von 8 cm) eingepflanzt.

Die Plfanzen werden auf einem Drehteller mit jeweils 100 ml einer acetonischen Lösung enthaltend 400 und 800 ppm Wirkstoff besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein Glaszylinder gestülpt und dieser mit einem Gaze-Deckel abgedeckt. Die Nymphen werden bis zum Erreichen des folgenden Entwicklungsstadiums über 10 Tage an der behandelten Pflanze gehalten. Die Bestimmung der Mortalität erfolgt 1, 4 und 8 Tage nach der Behandlung.

Verbindungen aus den Beispielen 1—6 und den Tabellen 1—4 zeigen gegen Nymphen von Nilaparvata lugens und von Laodelphax striatellus gute Wirksamkeit.

Beispiel 11

Wirkung gegen Nilaparvata lugens (ovizid) und Laodelphax striatellus

Der Test wird an wachsenden Reis-Pflanzen durchgeführt. Jeweils 4 Pflanzen (Dicke des Stengels 8 mm; Höhe ca. 20 cm) werden in Töpfe (Durchmesser von 8 cm) eingepflanzt.

Die Pflanzen werden auf einem drehteller mit jeweils 100 ml einer acetonischen Lösung enthaltend 400 ppm Wirkstoff besprüht. Nach dem Antrocknen des Spritzbelages wird jede Pflanze mit 3 adulten Weibchen besiedelt. Um die Tiere am Entweichen zu hindern, wird über jede besiedelte Pflanze ein Glaszylinder gestülpt und dieser mit einem Gaze-Deckel abgedeckt. Die Weibchen bleiben 4 Tage zur Eiablage auf der behandelten Pflanze und werden dann entfernt.

Etwa 8 Tage nach Besiedlung schlüpfen die jungen Zikaden, und es erfolgt die Auswertung. Aus dem Vergleich der Anzahl geschlüpfter Larven auf den behandelten Pflanzen zu den auf den unbehandelten Kontrollpflanzen geschlüpften Tieren wird die Mortalität ermittelt.

Verbindungen aus den Beispielen 1—6 und den Tabellen 1—4 zeigen in obigem Test eine über 80%ige ovizide Wirkung.

Wirkstoff-Formulierungen

Die Wirkstoffe der Formel I können beispielsweise wie folgt formuliert werden:

Granulat

5 Teile Wirkstoff,
0,25 Teile epoxydiertes Pflanzenöl,
0,25 Teile Cetylpolyglykoläther,
3,50 Teile Polyäthylenglykol
91 Teile Kaolin (Korngrösse 0,3—0,8 mm).

Die Aktivsubstanz wird mit dem epoxydierten Pflanzenöl in 6 Teilen Aceton gelöst, hierauf wird Polyäthylenglykol und Cetylpolyglykoläther zugesetzt. Die so erhaltene Lösung wird auf Koalin aufgesprüht und anschliessend das Aceton im Vakuum verdampft. Solche Granulate können dem Viehfutter zugesetzt werden.

Stäubemittel

5 Gew.-Teile feingemahlener Wirkstoff werden mit
2 Gew.-Teilen einer gefällten Kieselsäure und
93 Gew.-Teilen Talk intensiv gemischt.

Der Wirkstoff wird mit den Trägerstoffen homogen gemischt und vermahlen. Das Stäubemittel kann nicht nur äusserlich, sondern auch als Zusatz zum Viehfütter angewendet werden.

Spritzpulver

5 bis 30 Gew.-Teile Wirkstoff werden in einer Mischapparatur mit
5 Gew.-Teilen eines aufsaugenden Trägermaterials (Kieselsäure K 320 oder Wessalon 5) und
55 bis 80 Gew.-Teilen eines Trägermaterials [Bolus alba oder Kaolin (B 24)] und einem Dispergiermittelgemisch, bestehend aus
5 Gew.-Teilen eines Na-Laurylsulfonats und
5 Gew.-Teilen eines Alkyl-aryl-polyglykoläthers, intensiv vermischt.

Diese Mischung wird in einer Stift- oder Luftstrahlmühle bis auf 5—15 µm gemahlen. Das so erhaltene Spritzpulver gibt in Wasser eine gute Suspension.

Emulsionskonzentrat

20 Gew.-Teile Wirkstoff werden in
70 Gew.-Teilen Xylol gelöst und mit
10 Gew.-Teilen eines Emulgiermittels, bestehend aus einem Gemisch eines Alkylphenylpolyglykoläthers und dem Calciumsalz der Dodecylbenzolsulfonsäure, versetzt.

Das Emulsionskonzentrat kann in beliebigem Verhältnis mit Wasser versetzt werden und bildet dabei eine milchige Emulsion, die den Viehtränken zugesetzt werden kann.

Aufguss-Lösung (pour-on)

| | |
|---|---|
| Wirksubstanz | 30,00 g |
| Natrium Dioctylsulfosuccinat | 3,00 g |
| Benzylalkohol | 35,46 g |
| Aethylenglykol-monomethyläther | 35,46 g |
| | 103,92 g = 100 ml |

Die Wirksubstanz wird in dem grössten Teil des Gemisches der beiden Lösungsmittel unter kräftigem Rühren gelöst. Anschliessend wird das Natrium-dioctylsulfosuccinat, eventuell unter Erwärmen, gelöst und schliesslich mit dem restlichen Lösungsmittelgemisch aufgefüllt.

**Patentansprüche für die Vertragsstaaten: BE DE FR IT NL SE CH LI**

1. Verbindungen der Formel I

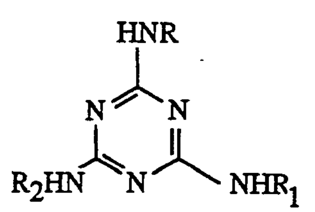

(I)

in welcher

R $C_1$—$C_4$-Alkyl oder $C_3$—$C_6$-Cycloalkyl,

$R_1$ Wasserstoff oder gegebenenfalls durch Halogen oder $C_1$—$C_4$-Alkoxy substituiertes Alkanoyl mit 2 bis 12 Kohlenstoffatomen oder $C_3$—$C_6$-Cycloalkyl-carbonyl und

$R_2$ gegebenenfalls durch Halogen oder $C_1$—$C_4$-Alkoxy substituiertes Alkanoyl mit 2 bis 12 Kohlenstoffatomen oder $C_3$—$C_6$-Cycloalkyl-carbonyl bedeuten, sowie deren Säureadditionssalze, mit der Massgabe, dass

a) wenn R Methyl und $R_1$ Wasserstoff ist, $R_2$ nicht Acetyl ist, und

b) wenn R $C_1$—$C_4$-Alkyl und $R_1$ Alkanoyl mit 2 bis 4 Kohlenstoffatomen ist, $R_2$ nicht Alkanoyl mit 2 bis 4 Kohlenstoffatomen ist.

2. Verbindungen der Formel I

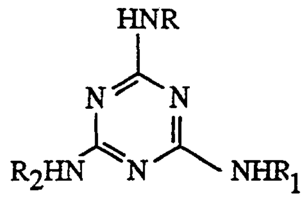

(I)

in welcher

R $C_1$—$C_4$-Alkyl oder $C_3$—$C_6$-Cycloalkyl,

$R_1$ Wasserstoff oder gegebenenfalls durch Halogen substituiertes Alkanoyl mit 2 bis 12 Kohlenstoffatomen oder $C_3$—$C_6$-Cycloalkyl-carbonyl und

$R_2$ gegebenenfalls durch Halogen substituiertes Alkanoyl mit 2 bis 12 Kohlenstoffatomen oder $C_3$—$C_6$-Cycloalkyl-carbonyl bedeuten, sowie deren Säureadditionssalze, mit der Massgabe, dass a) wenn R Methyl und $R_1$ Wasserstoff ist, $R_2$ nicht Acetyl ist, und b) wenn R $C_1$—$C_4$-Alkyl und $R_1$ Alkanoyl mit 2 bis 4 Kohlenstoffatomen ist, $R_2$ nicht Alkanoyl mit 2 bis 4 Kohlenstoffatomen ist.

3. Verbindungen der Formel I gemäss Anspruch 1, in welcher

R $C_1$—$C_4$-Alkyl oder $C_3$—$C_6$-Cycloalkyl,

$R_1$ Wasserstoff oder gegebenenfalls durch $C_1$—$C_4$-Alkoxy substituiertes Alkanoyl mit 2 bis 12 Kohlenstoffatomen oder $C_3$—$C_6$-Cycloalkyl-carbonyl und

$R_2$ gegebenenfalls durch $C_1$—$C_4$-Alkoxy substituiertes Alkanoyl mit 2 bis 12 Kohlenstoffatomen oder $C_3$—$C_6$-Cycloalkyl-carbonyl bedeuten, sowie deren Säureadditionssalze, mit der Massgabe, dass a) wenn R Methyl und $R_1$ Wasserstoff ist, $R_2$ nicht Acetyl ist, und b) wenn R $C_1$—$C_4$-Alkyl und $R_1$ Alkanoyl mit 2 bis 4 Kohlenstoffatomen ist, $R_2$ nicht Alkanoyl mit 2 bis 4 Kohlenstoffatomen ist.

4. Verbindungen der Formel I gemäss Anspruch 2, in welcher

R Cyclopropyl oder iso-Propyl,

$R_1$ Wasserstoff oder gegebenenfalls durch Chlor substituiertes Alkanoyl mit 2 bis 12 Kohlenstoffatomen oder $C_3$—$C_6$-Cycloalkyl-carbonyl und

$R_2$ gegebenenfalls durch Chlor substituiertes Alkanoyl mit 2 bis 12 Kohlenstoffatomen oder $C_3$—$C_6$-Cycloalkyl-carbonyl bedeuten, sowie deren Säureadditionssalze, mit der Massgabe, dass, wenn R Isopropyl und $R_1$ Alkanoyl mit 2 bis 4 Kohlenstoffatomen ist, $R_2$ nicht Alkanoyl mit 2 bis 4 Kohlenstoffatomen ist.

5. Verbindungen der Formel I gemäss Anspruch 2, in welchen

R Cyclopropyl oder iso-Propyl,

$R_1$ Wasserstoff und

$R_2$ gegebenenfalls durch Chlor substituiertes Alkanoyl mit 2 bis 12 Kohlenstoffatomen oder $C_3$—$C_6$-Cycloalkyl-carbonyl bedeuten, sowie deren Säureadditionssalze.

6. Verbindungen der Formel I gemäss Anspruch 2, in welchen

R Cyclopropyl,

$R_1$ Wasserstoff und

$R_2$ Alkanoyl mit 2 bis 12 Kohlenstoffatomen oder $C_3$—$C_6$-Cycloalkyl-carbonyl bedeuten, sowie deren Säureadditionssalze.

7. 2-Cyclopropylamino-4-acetylamino-6-amino-1,3,5-triazin gemäss Anspruch 2.

8. 2-Cyclopropylamino-4-methoxyacetylamino-6-amino-1,3,5-triazin gemäss Anspruch 3.

9. 2-Cyclopropylamino-4-propanoylamino-6-amino-1,3,5-triazin gemäss Anspruch 2.

10. 2-Cyclopropylamino-4-butanoylamino-6-amino-1,3,5-triazin gemäss Anspruch 2.

11. 2-Cyclopropylamino-4-(2-methylpropanoylamino)-6-amino-1,3,5-triazin gemäss Anspruch 2.

12. Verfahren zur Herstellung einer Verbindung der Formel I

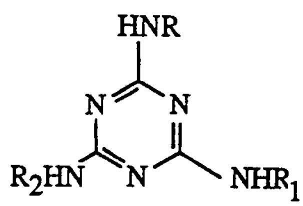

in welcher

R $C_1$—$C_4$-Alkyl oder $C_3$—$C_6$-Cycloalkyl,

$R_1$ Wasserstoff oder gegebenenfalls durch Halogen oder $C_1$—$C_4$-Alkoxy substituiertes Alkanoyl mit 2

bis 12 Kohlenstoffatomen oder $C_3$—$C_6$-Cycloalkyl-carbonyl und

$R_2$ gegebenenfalls durch Halogen oder $C_1$—$C_4$-Alkoxy substituiertes Alkanoyl mit 2 bis 12 Kohlenstoffatomen oder $C_3$—$C_6$-Cycloalkyl-carbonyl bedeuten, sowie deren Säureadditionssalzen, dadurch gekennzeichnet, dass man eine Verbindung der Formel II

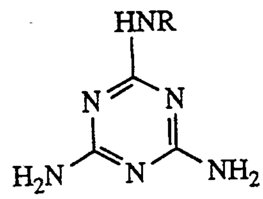

(II)

mit einer Verbindung der Formel IIIa

$R_2'$—CO—Hal (IIIa)

oder der Formel IIIa'

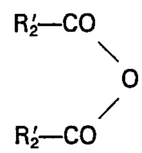

(IIIa')

zu einer Verbindung der Formel Ia

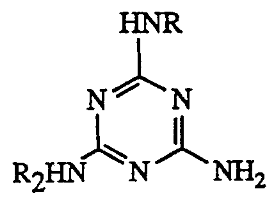

(Ia)

und gegebenenfalls eine Verbindung der Formel Ia mit einer Verbindung der Formel IIIb

$R_1''$—CO—Hal (IIIb)

oder der Formel IIIb'

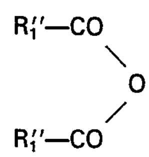

(IIIb')

zu einer Verbindung der Formel Ib

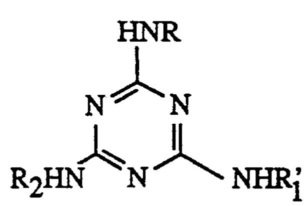

(Ib)

in inerten Lösungs oder Verdünnungsmitteln in Gegenwart einer Base bei Temperaturen von 0 bis 120°C umsetzt, in welchen R $C_1$—$C_4$-Alkyl oder $C_3$—$C_6$-Cycloalkyl,

$R_1'$ und $R_2$ unabhängig voneinander gegebenenfalls durch Halogen oder $C_1$—$C_4$-Alkoxy substituiertes Alkanoyl mit 2 bis 12 Kohlenstoffatomen oder $C_3$—$C_6$-Cycloalkyl-carbonyl und $R_1''$ und $R_2'$ unabhängig voneinander gegebenenfalls durch Halogen oder $C_1$—$C_4$-Alkoxy substituiertes $C_1$—$C_{11}$-Alkyl oder $C_3$—$C_6$-Cycloalkyl darstellen und Hal Halogen bedeutet.

13. Verfahren gemäss Anspruch 12, dadurch gekennzeichnet, dass die Umsetzungstemperatur 40° bis 80°C beträgt.

14. Schädlingsbekämpfungsmittel, welches als aktive Komponente eine Verbindung gemäss den Ansprüchen 1 bis 11 zusammen mit Träger- und/oder weiteren Zusatzstoffen enthält.

15. Verfahren zur Bekämpfung von tierparasitären Schadinsekten oder Ektoparasiten auf oder in ihren

Lebensräumen mit Ausnahme menschlicher oder tierischer Körper, dadurch gekennzeichnet, dass eine Verbindung der Formel I gemäss den Ansprüchen 1 bis 11 verwendet wird.

16. Verbindung der Formel I gemäss den Ansprüchen 1 bis 11 zur Anwendung bei der Bekämpfung von tierparasitären Schadinsekten oder Ektoparasiten.

17. Verwendung einer Verbindung der Formel I gemäss den Ansprüchen 1 bis 11 zur Herstellung eines Arzneimittels zur Bekämpfung von tierparasitären Schadinsekten oder Ektoparasiten.

18. Verfahren zur Bekämpfung von Schadinsekten in den ausgeschiedenen Fäkalien von Nutztieren, dadurch gekennzeichnet, dass man den Nutztieren eine Verbindung der Formel I gemäss Anspruch 1 oral appliziert.

**Patentansprüche für den Vertragsstaat: AT**

1. Verfahren zur Herstellung einer Verbindung der Formel I

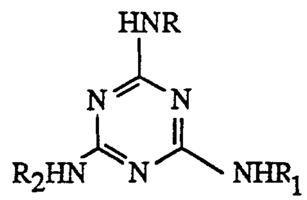

(I)

in welcher

R $C_1$—$C_4$-Alkyl oder $C_3$—$C_6$-Cycloalkyl,

$R_1$ Wasserstoff oder gegebenenfalls durch Halogen oder $C_1$—$C_4$-Alkoxy substituiertes Alkanoyl mit 2 bis 12 Kohlenstoffatomen oder $C_3$—$C_6$-Cycloalkyl-carbonyl und

$R_2$ gegebenenfalls durch Halogen oder $C_1$—$C_4$-Alkoxy substituiertes Alkanoyl mit 2 bis 12 Kohlenstoffatomen oder $C_3$—$C_6$-Cycloalkyl-carbonyl bedeuten, sowie deren Säureadditionssalzen, dadurch gekennzeichnet, dass man eine Verbindung der Formel II

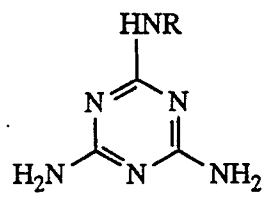

(II)

mit einer Verbindung der Formel IIIa

$$R_2'—CO—Hal \quad (IIIa)$$

oder der Formel IIIa'

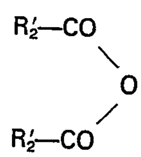

(IIIa')

zu einer Verbindung der Formel Ia

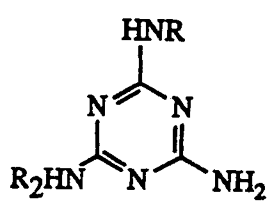

(Ia)

und gegebenenfalls eine Verbindung der Formel Ia mit einer Verbindung der Formel IIIb

$$R_1''—CO—Hal \quad (IIIb)$$

oder der Formel IIIb'

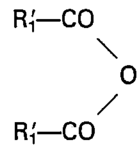

(IIIb')

zu einer Verbindung der Formel Ib

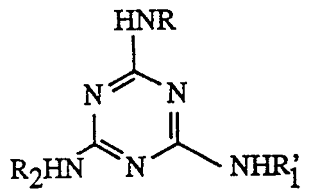

(Ib)

in welchen R $C_1$—$C_4$-Alkyl oder $C_3$—$C_6$-Cycloalkyl, $R_1'$ und $R_2$ gegebenenfalls durch Halogen oder $C_1$—$C_4$-Alkoxy substituiertes Alkanoyl mit 2 bis 12 Kohlenstoffatomen oder $C_3$—$C_6$-Cycloalkyl-carbonyl und $R_1''$ und $R_2'$ unabhängig voneinander gegebenenfalls durch Halogen und $C_1$—$C_4$-Alkoxy substituiertes $C_1$—$C_{11}$-Alkyl oder $C_3$—$C_6$-Cycloalkyl darstellen und Hal Halogen bedeutet, in inerten Lösungs- oder Verdünnungsmitteln in Gegenwart einer Base bei Temperaturen von 0 bis 120°C umsetzt.

2. Verfahren gemäss Anspruch 1, dadurch gekennzeichnet, dass die Umsetzungstemperatur 40° bis 80°C beträgt.

3. Verfahren gemäss den Ansprüchen 1 und 2 zur Herstellung von Verbindungen der Formel I, in welcher R $C_1$—$C_4$-Alkyl oder $C_3$—$C_6$-Cycloalkyl, $R_1$ Wasserstoff oder gegebenenfalls durch Halogen substituiertes Alkanoyl mit 2 bis 12 Kohlenstoffatomen oder $C_3$—$C_6$-Cycloalkyl-carbonyl und $R_2$ gegebenenfalls durch Halogen substituiertes Alkanoyl mit 2 bis 12 Kohlenstoffatomen oder $C_3$—$C_6$-Cycloalkyl-carbonyl bedeuten, sowie deren Säureadditionssalzen.

4. Verfahren gemäss den Ansprüchen 1 und 2 zur Herstellung von Verbindungen der Formel I, in welcher R $C_1$—$C_4$-Alkyl oder $C_3$—$C_6$-Cycloalkyl, $R_1$ Wasserstoff oder gegebenenfalls durch $C_1$—$C_4$-Alkoxy substituiertes Alkanoyl mit 2 bis 12 Kohlenstoffatomen oder $C_3$—$C_6$-Cycloalkyl-carbonyl und $R_2$ gegebenenfalls durch $C_1$—$C_4$-Alkoxy substituiertes Alkanoyl mit 2 bis 12 Kohlenstoffatomen oder $C_3$—$C_6$-Cycloalkyl-carbonyl bedeuten, sowie deren Säureadditionssalzen.

5. Verfahren gemäss den Ansprüchen 1 und 2 zur Herstellung von Verbindungen der Formel I, in welcher R Cyclopropyl oder iso-Propyl, $R_1$ Wasserstoff oder gegebenenfalls durch Chlor substituiertes Alkanoyl mit 2 bis 12 Kohlenstoffatomen oder $C_3$—$C_6$-Cycloalkyl-carbonyl und $R_2$ gegebenenfalls durch Chlor substituiertes Alkanoyl mit 2 bis 12 Kohlenstoffatomen oder $C_3$—$C_6$-Cycloalkyl-carbonyl bedeuten, sowie deren Säureadditionssalzen.

6. Verfahren gemäss den Ansprüchen 1 und 2 zur Herstellung von Verbindungen der Formel I, in welchen R Cyclopropyl oder iso-Propyl, $R_1$ Wasserstoff und $R_2$ gegebenenfalls durch Chlor substituiertes Alkanoyl mit 2 bis 12 Kohlenstoffatomen oder $C_3$—$C_6$-Cycloalkyl-carbonyl bedeuten, sowie deren Säureadditionssalzen.

7. Verfahren gemäss den Ansprüchen 1 und 2 zur Herstellung von Verbindungen der Formel I, in welchen R Cyclopropyl, $R_1$ Wasserstoff und $R_2$ Alkanoyl mit 2 bis 12 Kohlenstoffatomen oder $C_3$—$C_6$-Cycloalkyl-carbonyl bedeuten.

8. Verfahren gemäss den Ansprüchen 1 und 2 zur Herstellung von 2-Cyclopropylamino-4-acetylamino-6-amino-1,3,5-triazin.

9. Verfahren gemäss den Ansprüchen 1 und 2 zur Herstellung von 2-Cyclopropylamino-4-methoxyacetylamino-6-amino-1,3,5-triazin.

10. Verfahren gemäss den Ansprüchen 1 und 2 zur Herstellung von 2-Cyclopropylamino-4-propanoylamino-6-amino-1,3,5-triazin.

11. Verfahren gemäss den Ansprüchen 1 und 2 zur Herstellung von 2-Cyclopropylamino-4-butanoylamino-6-amino-1,3,5-triazin.

12. Verfahren gemäss den Ansprüchen 1 und 2 zur Herstellung von 2-Cyclopropylamino-4-(2-methylpropanoylamino)-6-amino-1,3,5-triazin.

13. Shädlingsbekämpfungsmittel, welches als aktive Komponente eine Verbindung hergestellt gemäss den Ansprüchen 1 bis 12 zusammen mit Träger- und/oder weiteren Zusatzstoffen enthält.

14. Verfahren zur Bekämpfung von tierparasitären Schadinsekten oder Ektoparasiten auf oder in ihren Lebensräumen mit Ausnahme menschlicher oder tierischer Körper, dadurch gekennzeichnet, dass eine Verbindung der Formel I gemäss den Ansprüchen 1 bis 12 verwendet wird.

15. Verbindung der Formel I gemäss den Ansprüchen 1 bis 12 zur Anwendung bei der Bekämpfung von tierparasitären Schadinsekten oder Ektoparasiten.

16. Verwendung einer Verbindung der Formel I gemäss den Ansprüchen 1 bis 12 zur Herstellung eines Arzneimittels zur Bekämpfung von tierparasitären Schadinsekten oder Ektoparasiten.

17. Verfahren zur Bekämpfung von Schadinsekten in den ausgeschiedenen Fäkalien von Nutztieren,

dadurch gekennzeichnet, dass man den Nutztieren eine Verbindung der Formel I gemäss Anspruch 1 oral appliziert.

**Revendications pour les Etats contractants: BE DE FR IT NL SE CH LI**

1. Composés de formule I

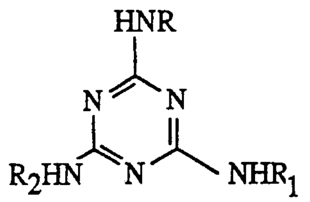

(I)

dans laquelle

R représente un groupe alkyle en $C_1$—$C_4$ ou cycloalkyle en $C_3$—$C_6$,

$R_1$ représente l'hydrogène ou un groupe alcanoyle en $C_2$—$C_{12}$ ou (cycloalkyle en $C_3$—$C_6$)-carbonyle éventuellement substitué par des halogènes ou des groupes alcoxy en $C_1$—$C_4$, et

$R_2$ représente un groupe alcanoyle en $C_2$—$C_{12}$ ou (cycloalkyle en $C_3$—$C_6$)-carbonyle éventuellement substitué par des halogènes ou des groupes alcoxy en $C_1$—$C_4$, et leurs sels formés par addition avec des acides, sous réserve que:

a) lorsque R représente un groupe méthyle et $R_1$ l'hydrogène, $R_2$ ne peut représenter un groupe acétyle, et

b) lorsque R représente un groupe alkyle en $C_1$—$C_4$ et $R_1$ un groupe alcanoyle en $C_2$—$C_4$, $R_2$ ne peut représenter un groupe alcanoyle en $C_2$—$C_4$.

2. Composés de formule I

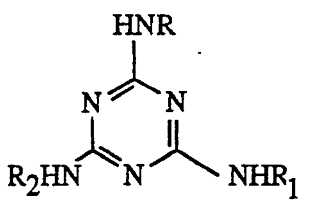

(I)

dans laquelle

R représente un groupe alkyle en $C_1$—$C_4$ ou cycloalkyle en $C_3$—$C_6$,

$R_1$ représente l'hydrogène ou un groupe alcanoyle en $C_2$—$C_{12}$ ou (cycloalkyle en $C_3$—$C_6$)-carbonyle éventuellement substitué par des halogènes, et

$R_2$ représente un groupe alcanoyle en $C_2$—$C_{12}$ ou (cycloalkyle en $C_3$—$C_6$)-carbonyle éventuellement substitué par des halogènes, et leurs sels formés par addition avec des acides, sous réserve que a) lorsque R représente un groupe méthyle et $R_1$ l'hydrogène, $R_2$ ne peut représenter un groupe acétyle et b) lorsque R représente un groupe alkyle en $C_1$—$C_4$ et $R_1$ un groupe alcanoyle en $C_2$—$C_4$, $R_2$ ne peut représenter un groupe alcanoyle en $C_2$—$C_4$.

3. Composés de formule I selon la revendication 1, dans lesquels

R représente un groupe alkyle en $C_1$—$C_4$ ou cycloalkyle en $C_3$—$C_6$,

$R_1$ représente l'hydrogène ou un groupe alcanoyle en $C_2$—$C_{12}$ ou (cycloalkyle en $C_3$—$C_6$)-carbonyle éventuellement substitué pa des groupes alcoxy en $C_1$—$C_4$, et

$R_2$ représente un groupe alcanoyle en $C_2$—$C_{12}$ ou (cycloalkyle en $C_3$—$C_6$)-carbonyle éventuellement substitué par des groupes alcoxy en $C_1$—$C_4$, et leurs sels formés par addition avec des acides, sous réserve que a) lorsque R représente un groupe méthyle et $R_1$ l'hydrogène, $R_2$ ne peut représenter un groupe acétyle, et b) lorsque R représente un groupe alkyle en $C_1$—$C_4$ et $R_1$ un groupe alcanoyle en $C_2$—$C_4$, $R_2$ ne peut représenter un groupe alcanoyle en $C_2$—$C_4$.

4. Composés de formule I selon la revendication 2, dans lesquels

R représente un groupe cyclopropyle ou isopropyle,

$R_1$ représente l'hydrogène ou un groupe alcanoyle en $C_2$—$C_{12}$ ou (cycloalkyle en $C_3$—$C_6$)-carbonyle éventuellement substitué par le chlore, et

$R_2$ représente un groupe alcanoyle en $C_2$—$C_{12}$ ou (cycloalkyle en $C_3$—$C_6$)-carbonyle éventuellement substitué par le chlore, et leurs sels formés par addition avec des acides, sous réserve que lorsque R représente un groupe isopropyle et $R_1$ un groupe alcanoyle en $C_2$—$C_4$, $R_2$ ne peut représenter un groupe alcanoyle en $C_2$—$C_4$.

5. Composés de formule I selon la revendication 2, dans lesquels

R représente un groupe cyclopropyle ou isopropyle,

$R_1$ représente l'hydrogène, et

$R_2$ représente un groupe alcanoyle en $C_2$—$C_{12}$ ou (cycloalkyle en $C_3$—$C_6$)-carbonyle éventuellement substitué par le chlore, et leurs sels formés par addition avec des acides.

6. Composés de formule I selon la revendication 2, dans lesquels

R représente un groupe cyclopropyle,

$R_1$ représente l'hydrogène, et

$R_2$ un groupe alcanoyle en $C_2$—$C_{12}$ ou (cycloalkyle en $C_3$—$C_6$)-carbonyle, et leurs sels formés par addition avec des acides.

7. La 2-cyclopropylamino-4-acétylamino-6-amino-1,3,5-triazine selon la revendication 2.

8. La 2-cyclopropylamino-4-méthoxyacétylamino-6-amino-1,3,5-triazine selon la revendication 3.

9. La 2-cyclopropylamino-4-propanoylamino-6-amino-1,3,5-triazine selon la revendication 2.

10. La 2-cyclopropylamino-4-butanoylamino-6-amino-1,3,5-triazine selon la revendication 2.

11. La 2-cyclopropylamino-4-(2-méthylpropanoylamino)-6-amino-1,3,5-triazine selon la revendication 2.

12. Procédé de préparation d'un composé de formule I

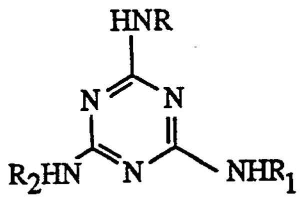

dans laquelle

R représente un groupe alkyle en $C_1$—$C_4$ ou cycloalkyle en $C_3$—$C_6$,

$R_1$ représente l'hydrogène ou un groupe alcanoyle en $C_2$—$C_{12}$ ou (cycloalkyle en $C_3$—$C_6$)-carbonyle éventuellement substitué par des halogènes ou des groupes alcoxy en $C_1$—$C_4$, et

$R_2$ représente un groupe alcanoyle en $C_2$—$C_{12}$ ou (cycloalkyle en $C_3$—$C_6$)-carbonyle éventuellement substitué par des halogènes ou des groupes alcoxy en $C_1$—$C_4$, et de leurs sels formés par addition avec des acides, caractérisé en ce que l'on fait réagir un composé de formule II

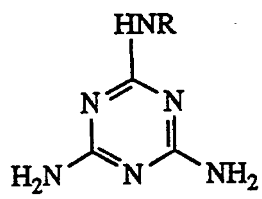

(II)

avec un composé de formule IIIa

$R_2'$—CO—Hal (IIIa)

ou de formule IIIa'

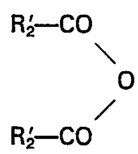

(IIIa')

ce qui donne un composé de formule Ia

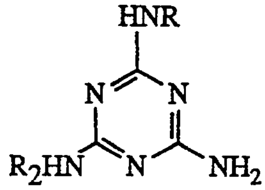

(Ia)

qu'on fait réagir éventuellement avec un composé de formule IIIb

$R_1''$—CO—Hal (IIIb)

ou de formule IIIb'

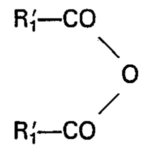

(IIIb')

ce qui donne un composé de formule Ib

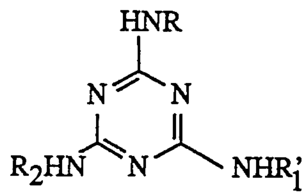

(Ib)

dans des solvants ou diluants inertes, en présence d'une base, à des températures de 0 à 120°C, les symboles des formules ci-dessus ayant les significations suivantes: R représente un groupe alkyle en $C_1$—$C_4$ ou cycloalkyle en $C_3$—$C_6$, $R_1'$ et $R_2$ représentent chacun, indépendamment l'un de l'autre, un groupe alcanoyle en $C_2$—$C_{12}$ ou (cycloalkyle en $C_3$—$C_6$)-carbonyle éventuellement substitué par des halogènes ou des groupes alcoxy en $C_1$—$C_4$ et $R_1''$ et $R_2'$ représentent chacun, indépendamment l'un de l'autre, un groupe alkyle en $C_1$—$C_{11}$ ou cycloalkyle en $C_3$—$C_6$ éventuellement substitué par des halogènes ou des groupes alcoxy en $C_1$—$C_4$, et Hal représente un halogène.

13. Procédé selon la revendication 12, caractérisé en ce que la température de réaction est de 40 à 80°C.

14. Produit pesticide contenant en tant que composant actif un composé selon les revendications 1 à 11 avec des véhicules et/ou d'autres additifs.

15. Procédé pour combattre les insectes nuisibles ou ectoparasites parasitant les animaux sur ou dans leur habitat, à l'exception du corps humain ou animal, caractérisé en ce que l'on utilise un composé de formule I selon les revendications 1 à 11.

16. Composé de formule I selon les revendications 1 à 11, pour l'utilisation dans la lutte contre les insectes nuisibles ou ectoparasites parasitant les animaux.

17. Utilisation d'un composé de formule I selon les revendications 1 à 11, pour la préparation d'un médicament servant lui-même à combattre les insectes nuisibles ou ectoparasites parasitant les animaux.

18. Procédé pour combattre les insectes nuisibles dans les matières fécales excrétées par les animaux utiles, caractérisé en ce que l'on administre aux animaux utiles un composé de formule I selon la revendication 1 par voie orale.

**Revendications pour l'Etat contractant: AT**

1. Procédé de préparation d'un composé de formule I

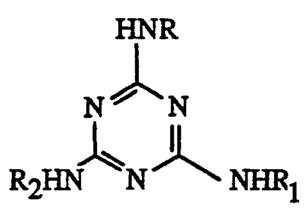

(I)

dans laquelle

R représente un groupe alkyle en $C_1$—$C_4$ ou cycloalkyle en $C_3$—$C_6$,

$R_1$ représente l'hydrogène ou un groupe alcanoyle en $C_2$—$C_{12}$ ou (cycloalkyle en $C_3$—$C_6$)-carbonyle éventuellement substitué par des halogènes ou des groupes alcoxy en $C_1$—$C_4$, et

$R_2$ représente un groupe alcanoyle en $C_2$—$C_{12}$ ou (cycloalkyle en $C_3$—$C_6$)-carbonyle éventuellement substitué par des halogènes ou des groupes alcoxy en $C_1$—$C_4$, et de leurs sels formés par addition avec des acides, caractérisé en ce que l'on fait réagir un composé de formule II

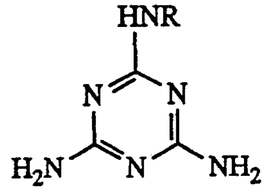

(II)

avec un composé de formule IIIa

$R_2'—CO—Hal$ (IIIa)

ou de formule IIIa'

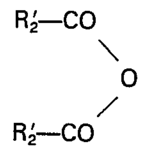

(IIIa')

ce qui donne un composé de formule Ia

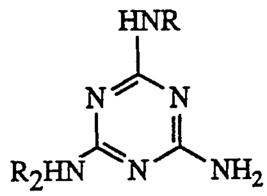

(Ia)

qu'on fait réagir éventuellement avec un composé de formule IIIb

$R_1''—CO—Hal$ (IIIb)

ou de formule IIIb'

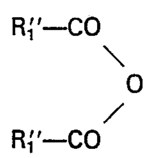

(IIIb')

ce qui donne un composé de formule Ib

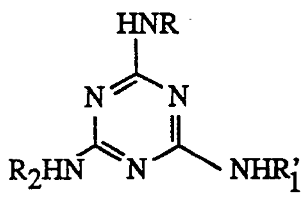

(Ib)

les symboles des formules ci-dessus ayant les significations suivantes: R représente un groupe alkyle en $C_1—C_4$ ou cycloalkyle en $C_3—C_6$, $R_1'$ et $R_2$ représentent des groupes alcanoyle en $C_2—C_{12}$ ou (cycloalkyle en $C_3—C_6$)-carbonyle éventuellement substitués par des halogènes ou des groupes alcoxy en $C_1—C_4$ et $R_1''$ et $R_1'$ représentent chacun, indépendamment l'un de l'autre, un groupe alkyle en $C_1—C_{11}$ ou cycloalkyle en $C_3—C_6$ éventuellement substitué par des halogènes ou des groupes alcoxy en $C_1—C_4$, et Hal représente un halogène, dans des solvants ou diluants inertes, en présence d'une base, à des températures de 0 à 120°C.

2. Procédé selon la revendication 1, caractérisé en ce que la température de réaction est de 40 à 80°C.

3. Procédé selon les revendications 1 et 2, pour la préparation de composés de formule I dans lesquels R représente un groupe alkyle en $C_1—C_4$ ou cycloalkyle en $C_3—C_6$, $R_1$ représente l'hydrogène ou un groupe alcanoyle en $C_2—C_{12}$ ou (cycloalkyle en $C_3—C_6$)-carbonyle éventuellement substitué par des halogènes et $R_2$ représente un groupe alkyle en $C_2—C_{12}$ ou (cycloalkyle en $C_3—C_6$)-carbonyle éventuellement substitué par des halogènes, et de leurs sels formés par addition avec des acides.

4. Procédé selon les revendications 1 et 2, pour la préparation de composés de formule I dans lesquels R représente un groupe alkyle en $C_1—C_4$ ou cycloalkyle en $C_3—C_6$, $R_1$ représente l'hydrogène ou un groupe alcanoyle en $C_2—C_{12}$ ou (cycloalkyle en $C_3—C_6$)-carbonyle éventuellement substitué par des groupes alcoxy en $C_1—C_4$, et $R_2$ représente un groupe alcanoyle en $C_2—C_{12}$ ou (cycloalkyle en $C_3—C_6$)-carbonyle éventuellement substitué par des groupes alcoxy en $C_1—C_4$, et de leurs sels formés par addition avec des acides.

5. Procédé selon les revendications 1 et 2, pour la préparation de composés de formule I dans lesquels R représente un groupe cyclopropyle ou isopropyle, $R_1$ représente l'hydrogène ou un groupe alcanoyle en $C_2—C_{12}$ ou (cycloalkyle en $C_3—C_6$)-carbonyle éventuellement substitué par le chlore et $R_2$ représente un groupe alcanoyle en $C_2—C_{12}$ ou (cycloalkyle en $C_3—C_6$)-carbonyle éventuellement substitué par le chlore, et de leurs sels formés par addition avec des acides.

6. Procédé selon les revendications 1 et 2, pour la préparation de composés de formule I dans lesquels R représente un groupe cyclopropyle ou isopropyle, $R_1$ représente l'hydrogène et $R_2$ un groupe alcanoyle

en $C_2$—$C_{12}$ ou (cycloalkyle en $C_3$—$C_6$)-carbonyle éventuellement substitué par le chlore, et de leurs sels formés par addition avec des acides.

7. Procédé selon les revendications 1 et 2, pour la préparation de composés de formule I dans lesquels R représente un groupe cyclopropyle, $R_1$ l'hydrogène et $R_2$ un groupe alcanoyle en $C_2$—$C_{12}$ ou (cycloalkyle en $C_3$—$C_6$)-carbonyle.

8. Procédé selon les revendications 1 et 2, pour la préparation de la 2-cyclopropylamino-4-acétylamino-6-amino-1,3,5-triazine.

9. Procédé selon les revendications 1 et 2, pour la préparation de la 2-cyclopropylamino-4-méthoxyacétylamino-6-amino-1,3,5-triazine.

10. Procédé selon les revendications 1 et 2, pour la préparation de la 2-cyclopropylamino-4-propanoylamino-6-amino-1,3,5-triazine.

11. Procédé selon les revendications 1 et 2, pour la préparation de la 2-cyclopropylamino-4-butanoylamino-6-amino-1,3,5-triazine.

12. Procédé selon les revendications 1 et 2, pour la préparation de la 2-cyclopropylamino-4-(2-méthylpropanoylamino)-6-amino-1,3,5-triazine.

13. Produit pesticide contenant en tant que composant actif un composé préparé selon les revendications 1 à 12 avec des véhicules et/ou d'autres additifs.

14. Procédé pour combattre les insectes nuisibles ou ectoparasites parasitant les animaux sur ou dans leur habitat à l'exception du corps humain ou animal, caractérisé en ce que l'on utilise un composé de formule I selon les revendications 1 à 12.

15. Composé de formule I selon les revendications 1 à 12, pour l'utilisation dans la lutte contre les insectes nuisibles ou ectoparasites parasitant les animaux.

16. Utilisation d'un composé de formule I selon les revendications 1 à 12 pour la préparation d'un médicament servant à combattre les insectes nuisibles ou ectoparasites parasitant les animaux.

17. Procédé pour combattre les insectes nuisibles dans les matières fécales excrétées par les animaux utiles, caractérisé en ce que l'on administre aux animaux utiles, par voie orale, un composé de formule I selon la revendication 1.

**Claims for the Contracting States: BE CH DE FR IT LI NL SE**

1. Compounds of the formula I

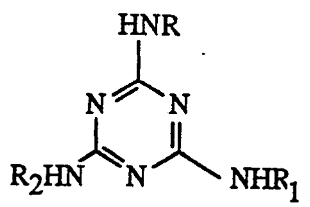

(I)

wherein

R is $C_1$—$C_4$-alkyl or $C_3$—$C_6$-cycloalkyl,

$R_1$ is hydrogen, $C_2$—$C_{12}$-alkanoyl which is unsubstituted or substituted by halogen or $C_1$—$C_4$-alkoxy, or is $C_3$—$C_6$-cycloalkylcarbonyl, and

$R_2$ is $C_2$—$C_{12}$-alkanoyl which is unsubstituted or substituted by halogen or $C_1$—$C_4$-alkoxy, or is $C_3$—$C_6$-cycloalkylcarbonyl, including the acid addition salts thereof, provided that

a) when R is methyl and $R_1$ is hydrogen then $R_2$ is not acetyl, and

b) when R is $C_1$—$C_4$-alkyl and $R_1$ is $C_2$—$C_4$-alkanoyl then $R_2$ is not $C_2$—$C_4$-alkanoyl.

2. Compounds of the formula I

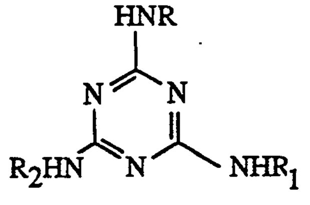

(I)

wherein

R is $C_1$—$C_4$-Alkyl or $C_3$—$C_6$-cycloalkyl,

$R_1$ is hydrogen, $C_2$—$C_{12}$-alkanoyl which is unsubstituted or substituted by halogen, or is $C_3$—$C_6$-cycloalkylcarbonyl, and

$R_2$ is $C_2$—$C_{12}$-alkanoyl which is unsubstituted or substituted by halogen, or is $C_3$—$C_6$-cycloalkyl carbonyl, including the acid addition salts thereof, provided that

a) when R is methyl and $R_1$ is hydrogen then $R_2$ is not acetyl, and

b) when R is $C_1$—$C_4$-alkyl and $R_1$ is $C_2$—$C_4$-alkanoyl then $R_2$ is not $C_2$—$C_4$-alkanoyl.

3. Compounds of the formula I according to claim 1, wherein

R is $C_1$—$C_4$-alkyl or $C_3$—$C_6$-cycloalkyl,

$R_1$ is hydrogen, $C_2$—$C_{12}$-alkanoyl which is unsubstituted or substituted by $C_1$—$C_4$-alkoxy, or is $C_3$—$C_6$-cycloalkylcarbonyl, and

$R_2$ is $C_2$—$C_{12}$-alkanoyl which is unsubstituted or substituted by $C_1$—$C_4$-alkoxy, or is $C_3$—$C_6$-cycloalkylcarbonyl, including the acid addition salts thereof, provided that

a) when R is methyl and $R_1$ is hydrogen then $R_2$ is not acetyl, and

b) when R is $C_1$—$C_4$-alkyl and $R_1$ is $C_2$—$C_4$-alkanoyl then $R_2$ is not $C_2$—$C_4$-alkanoyl.

4. Compounds of the formula I according to claim 2, wherein

R is cyclopropyl or isopropyl,

$R_1$ is hydrogen, $C_2$—$C_{12}$-alkanoyl which is unsubstituted or substituted by chlorine, or is $C_3$—$C_6$-cycloalkylcarbonyl, and

$R_2$ is $C_2$—$C_{12}$-alkanoyl which is unsubstituted or substituted by chlorine, or is $C_3$—$C_6$-cycloalkylcarbonyl, including the acid addition salts thereof, provided that when R is isopropyl and $R_1$ is $C_2$—$C_4$-alkanoyl then $R_2$ is not $C_2$—$C_4$-alkanoyl.

5. Compounds of the formula I according to claim 2, wherein

R is cyclopropyl or isopropyl,

$R_1$ is hydrogen and

$R_2$ is $C_2$—$C_{12}$-alkanoyl which is unsubstituted or substituted by chlorine, or is $C_3$—$C_6$-cycloalkylcarbonyl, including the acid addition salts thereof.

6. Compounds of the formula I according to claim 2, wherein

R is cyclopropyl,

$R_1$ is hydrogen and

$R_2$ is $C_2$—$C_{12}$-alkanoyl or $C_3$—$C_6$-cycloalkylcarbonyl, including the acid addition salts thereof.

7. 2-Cyclopropylamino-4-acetylamino-6-amino-1,3,5-triazine according to claim 2.

8. 2-Cyclopropylamino-4-methoxyacetylamino-6-amino-1,3,5-triazine according to claim 3.

9. 2-Cyclopropylamino-4-propanoylamino-6-amino-1,3,5-triazine according to claim 2.

10. 2-Cyclopropylamino-4-butanoylamino-6-amino-1,3,5-triazine according to claim 2.

11. 2-Cyclopropylamino-4-(2-methylpropanoylamino)-6-amino-1,3,5-triazine according to claim 2.

12. Process for producing a compound of the formula I

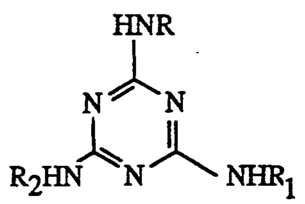

(I)

wherein

R is $C_1$—$C_4$-alkyl or $C_3$—$C_6$-cycloalkyl,

$R_1$ is hydrogen, $C_2$—$C_{12}$-alkanoyl which is unsubstituted or substituted by halogen or $C_1$—$C_4$-alkoxy, or is $C_3$—$C_6$-cycloalkylcarbonyl, and

$R_2$ is $C_2$—$C_{12}$-alkanoyl which is unsubstituted or substituted by halogen or $C_1$—$C_4$-alkoxy, or is $C_3$—$C_6$-cycloalkylcarbonyl, including the acid addition salts thereof, which process comprises reacting a compound of the formula II

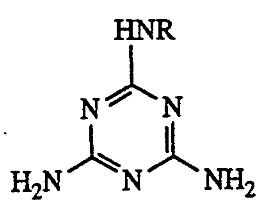

(II)

with a compound of the formula IIIa

$$R_2'—CO—Hal \qquad (IIIa)$$

or of the formula IIIa'

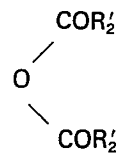

(IIIa')

to give a compound of the formula Ia

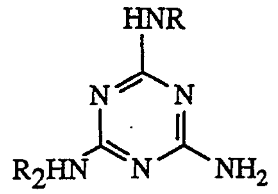

(Ia)

and optionally reacting a compound of the formula Ia in inert solvents or diluents, in the presence of a base and at a temperature range of between 0° and 120°C with a compound of the formula IIIb

$R_1''$—CO—Hal (IIIb)

or of the formula IIIb'

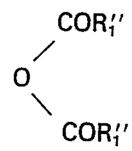

(IIIb')

to obtain a compound of the formula Ib

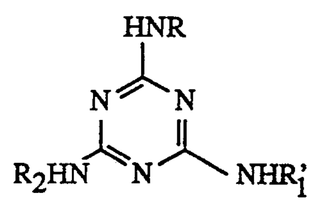

(Ib)

wherein

R is $C_1$—$C_4$-alkyl or $C_3$—$C_6$-cycloalkyl,

$R_1'$ and $R_2$ are independently of each other $C_2$—$C_{12}$-alkanoyl which is unsubstituted or substituted by halogen or $C_1$—$C_4$-alkoxy, or are $C_3$—$C_6$-cycloalkylcarbonyl, and

$R_1''$ and $R_2''$ are independently of each other $C_1$—$C_{11}$-alkyl which is unsubstituted or substituted by halogen or $C_1$—$C_4$-alkoxy, or are independently of each other $C_3$—$C_6$-cycloalkylcarbonyl, and

Hal is halogen.

13. Process according to claim 12, wherein the reaction temperature is in the range of between 40° and 80°C.

12. Pesticidal composition which contains as active ingredient a compound according to any one of claims 1 to 11, together with carriers and/or further additives.

15. Method of controlling harmful zooparasitic insects or ectoparasites which comprises applying an insecticidally or ectoparasiticidally effective amount of a compound of formula I according to any one of claims 1 to 11 to the insects or the ectoparasites or the locus thereof excepting the human or animal body.

16. Compound of formula I according to any one of claims 1 to 11 for the use in the control of harmful zooparasitic insects or ectoparasites.

17. Use of a compound of formula I according to any one of claims 1 to 11 for the production of a medicament for controlling harmful zooparasitic insects or ectoparasites.

18. Method of controlling harmful insects in the excreted faeces of productive animals which method comprises the oral application to said animals of a compound of formula I according to claim 1.

**Claims for the Contracting State: AT**

1. Process for producing a compound of the formula I

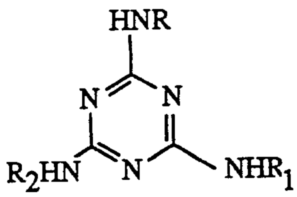

(I)

wherein

R is $C_1$—$C_4$-alkyl or $C_3$—$C_6$-cycloalkyl,

$R_1$ is hydrogen, $C_2$—$C_{12}$-alkanoyl which is unsubstituted or substituted by halogen or $C_1$—$C_4$-alkoxy, or is $C_3$—$C_6$-cycloalkylcarbonyl, and

$R_2$ is $C_2$—$C_{12}$-alkanoyl which is unsubstituted or substituted by halogen or $C_1$—$C_4$-alkoxy, or is $C_3$—$C_6$-cycloalkylcarbonyl, including the acid addition salts thereof, which process comprises reacting a compound of the formula II

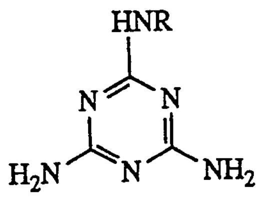

(II)

with a compound of the formula IIIa

$R_2'$—CO—Hal (IIIa)

or of the formula IIIa'

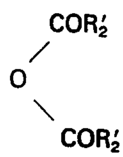

(IIIa')

to give a compound of the formula Ia

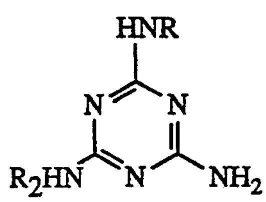

(Ia)

and optionally reacting a compound of the formula Ia in inert solvents or diluents, in the presence of a base and at a temperature range of between 0° and 120°C with a compound of the formula IIIb

$R_1''$—CO—Hal (IIIb)

or of the formula IIIb'

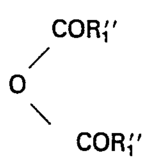

(IIIb')

to obtain a compound of the formula Ib

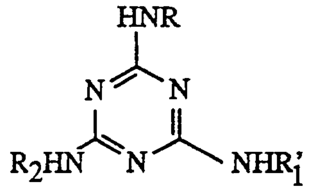

(Ib)

wherein

R is $C_1$—$C_4$-alkyl or $C_3$—$C_6$-cycloalkyl,

$R_1'$ and $R_2$ are independently of each other $C_2$—$C_{12}$-alkanoyl which is unsubstituted or substituted by halogen or $C_1$—$C_4$-alkoxy, or are $C_3$—$C_6$-cycloalkylcarbonyl, and

$R_1''$ and $R_2''$ are independently of each other $C_1$—$C_{11}$-alkyl which is unsubstituted or substituted by halogen or $C_1$—$C_4$-alkoxy, or are independently of each other $C_3$—$C_6$-cycloalkylcarbonyl, and

Hal is halogen.

2. Process according to claim 1, wherein the reaction temperature is in the range of between 40° and 80°C.

3. Process according to claims 1 and 2 to produce compounds of the formula I, wherein

R is $C_1$—$C_4$-alkyl or $C_3$—$C_6$-cycloalkyl,

$R_1$ is hydrogen, $C_2$—$C_{12}$-alkanoyl which is unsubstituted or substituted by halogen, or is $C_3$—$C_6$-cycloalkylcarbonyl, and

$R_2$ is $C_2$—$C_{12}$-alkanoyl which is unsubstituted or substituted by halogen, or is $C_3$—$C_6$-cycloalkylcarbonyl, including the acid addition salts thereof.

4. Process according to claims 1 and 2 to produce compounds of the formula I, wherein

R is $C_1$—$C_4$-alkyl or $C_3$—$C_6$-cycloalkyl,

$R_1$ is hydrogen, $C_2$—$C_{12}$-alkanoyl which is unsubstituted or substituted by $C_1$—$C_4$-alkoxy, or is $C_3$—$C_6$-cycloalkylcarbonyl, and

$R_2$ is $C_2$—$C_{12}$-alkanoyl which is unsubstituted or substituted by $C_1$—$C_4$-alkoxy, or is $C_3$—$C_6$-cycloalkylcarbonyl, including the acid salts thereof.

5. Process according to claims 1 and 2 to produce compounds of the formula I, wherein

R is cyclopropyl or isopropyl,

$R_1$ is hydrogen, $C_2$—$C_{12}$-alkanoyl which is unsubstituted or substituted by chlorine, or is $C_3$—$C_6$-cycloalkylcarbonyl, and

$R_2$ is $C_2$—$C_{12}$-alkanoyl which is unsubstituted or substituted by chlorine, or is $C_3$—$C_6$-cycloalkylcarbonyl, including the acid addition salts thereof.

6. Process according to claims 1 and 2 to produce compounds of the formula I, wherein

R is cyclopropyl or isopropyl,

$R_1$ is hydrogen and

$R_2$ is $C_2$—$C_{12}$-alkanoyl which is unsubstituted or substituted by chlorine, or is $C_3$—$C_6$-cycloalkylcarbonyl, including the acid addition salts thereof.

7. Process according to claims 1 and 2 to produce compounds of the formula I, wherein

R is cyclopropyl,

$R_1$ is hydrogen and

$R_2$ is $C_2$—$C_{12}$-alkanoyl or $C_3$—$C_6$-cycloalkylcarbonyl.

8. Process according to claims 1 and 2 to produce 2-cyclopropylamino-4-acetylamino-6-amino-1,3,5-triazine.

9. Process according to claims 1 and 2 to produce 2-cyclopropylamino-4-methoxyacetylamino-6-amino-1,3,5-triazine.

10. Process according to claims 1 and 2 to produce 2-cyclopropylamino-4-propanoylamino-6-amino-1,3,5-triazine.

11. Process according to claims 1 and 2 to produce 2-cyclopropylamino-4-butanoylamino-6-amino-1,3,5-triazine.

12. Process according to claims 1 and 2 to produce 2-cyclopropylamino-4-(2-methylpropanoylamino)-6-amino-1,3,5-triazine.

13. Pesticidal composition which contains as active ingredient a compound produced according to any one of claims 1 to 12, together with carriers and/or further additives.

14. Method of controlling harmful zooparasitic insects or ectoparasites which comprises applying an insecticidally or ectoparasiticidally effective amount of a compound of formula I according to any one of claims 1 to 12 to the insects or the ectoparasites or the locus thereof excepting the human or animal body.

15. Compound of formula I according to any one of claims 1 to 12 for the use in the control of harmful zooparasitic insects or ectoparasites.

16. Use of a compound of formula I according to any one of claims 1 to 12 for the production of a medicament for controlling harmful zooparasitic insects or ectoparasites.

17. Method of controlling harmful insects in the excreted faeces of productive animals which method comprises the oral application to said animals of a compound of formula I according to claim 1.